(19) Europäisches Patentamt
European Patent Office
Office européen des brevets

(11) Veröffentlichungsnummer: **0 216 345 B1**

(12) # EUROPÄISCHE PATENTSCHRIFT

(45) Veröffentlichungstag der Patentschrift: **19.02.92**

(21) Anmeldenummer: **86113036.7**

(22) Anmeldetag: **22.09.86**

(51) Int. Cl.5: **C07D 498/14**, C07D 401/06, C07D 401/12, C07D 401/04, C07D 471/14, C07D 513/14, A61K 31/495, A61K 31/535, A61K 31/55, A61K 31/47, //(C07D498/14,265:00,241:00, 221:00),(C07D498/14,265:00, 221:00,209:00)

(54) **Chinolinderivate.**

(30) Priorität: **24.09.85 CH 4120/85**
**07.08.86 CH 3177/86**

(43) Veröffentlichungstag der Anmeldung:
**01.04.87 Patentblatt 87/14**

(45) Bekanntmachung des Hinweises auf die Patenterteilung:
**19.02.92 Patentblatt 92/08**

(84) Benannte Vertragsstaaten:
**AT BE CH DE FR GB IT LI LU NL SE**

(56) Entgegenhaltungen:
**EP-A- 0 121 727**

(73) Patentinhaber: **F. HOFFMANN-LA ROCHE AG**
**Postfach 3255**
**CH-4002 Basel(CH)**

(72) Erfinder: **Jolidon, Synèse, Dr.**
**Weidenstr. 42**
**CH-4106 Therwil(CH)**
Erfinder: **Kompis, Ivan, Dr.**
**Goldentalweg 16**
**CH-4104 Oberwil(CH)**
Erfinder: **Locher, Rita, Dr.**
**Riehenring 71**
**CH-4058 Basel(CH)**
Erfinder: **Weiss, Ekkehard, Dr.**
**Unterer Baselblick 21B**
**W-7851 Inzlingen(DE)**
Erfinder: **Wyss, Pierre-Charles, Dr.**
**Unterwartweg 27**
**CH-4132 Muttenz(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

(74) Vertreter: **Lederer, Franz, Dr. et al**
**Patentanwalt Dr. Franz Lederer Lucile-Grahn-Strasse 22**
**W-8000 München 80(DE)**

**Beschreibung**

Die Erfindung betrifft neue Chinolinderivate, Verfahren zu ihrer Herstellung, neue in diesem Verfahren verwendbare Zwischenprodukte, sowie Arzneimittel auf der Basis der besagten Chinolinderivate oder Zwischenprodukte.

Diese Chinolinderivate sind Verbindungen der Formel

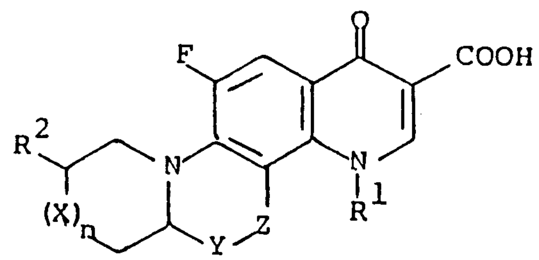

I

worin

| | | |
|---|---|---|
| | n | die Zahl 1 oder 0 |
| | X | eine Gruppe N-R |
| | R | Wasserstoff, $C_{1-4}$-Alkyl, $C_{2-4}$-Alkylen-N($R^a$,$R^b$) oder gegebenenfalls durch bis zu 3 Substituenten aus der Gruppe von Hydroxy, Halogen, $C_{1-4}$-Alkyl, $C_{1-4}$-Alkoxy oder Nitro ringsubstituiertes Benzyl |
| | Y | Methylen oder Aethylen |
| | Z | Methylen , 0 oder S |
| | $R^1$ | $C_{3-6}$-Cycloalkyl, N($R^c$,$R^d$), gegebenenfalls durch bis zu 3 Substituenten aus der Gruppe von Hydroxy, Halogen, $C_{1-4}$-Alkyl, $C_{1-4}$-Alkoxy oder Nitro substituiertes Phenyl oder gegebenenfalls fluoriertes $C_{1-4}$-Alkyl oder $C_{2-4}$-Alkenyl |
| | $R^2$ | Wasserstoff, $C_{1-4}$-Alkyl oder, falls n = 0 ist, auch OH oder N($R^e$,$R^f$) |
| | $R^a$ bis $R^g$ | Wasserstoff oder $C_{1-4}$-Alkyl sind, oder N($R^a$,$R^b$) ein 5- oder 6-gliedriger gesättigter Rest, gegebenenfalls mit einem zusätzlichen Heteroatom O oder N-$R^g$ ist, |

und physiologisch verträgliche Salze davon.

Beispiele von $C_{1-4}$-Alkyl- und $C_{2-4}$-Alkenylresten, die gerad- oder verzweigtkettig sein können, sind Methyl, Aethyl, Propyl, Isopropyl und Butyl bzw. Allyl und Vinyl. Beispiele für Aminoalkylenreste R sind Mono- oder Dimethylaminoäthyl. Beispiele für heterocyclische Reste -N($R^a$,$R^b$) sind Pyrrolidinyl, Piperidinyl, Morpholinyl und Piperazinyl. Beispiele für alkylierte Aminoreste $R^1$ oder $R^2$ sind Mono- und Dimethylamino. Ein Beispiel eines fluorierten Alkylrestes $R^1$ ist 2-Fluoräthyl. Beispiele für Cycloalkylreste $R^1$ sind Cyclopropyl und Cyclobutyl. Die Phenylreste $R^1$ und Benzylreste R können bis zu 3 Substituenten, wie Hydroxy, Halogen, z.B. Fluor oder Chlor. $C_{1-4}$-Alkyl oder -Alkoxy, z.B. Methyl oder Methoxy, oder Nitro enthalten.

Als physiologisch verträgliche Salze von Verbindungen der Formel I kommen Alkalimetall-, Erdalkalimetall- und ggf. substituierte Ammoniumsalze in Frage, sowie Additionssalze mit physiologisch verträglichen, starken anorganischen und organischen Säuren, wie Salzsäure, Schwefelsäure, Phosphorsäure, Methansulfonsäure oder p-Toluolsulfonsäure.

Die erfindungsgemässen Chinolinderivate enthalten mindestens ein asymmetrisches C-Atom und können somit als Enantiomere, als Diastereomere oder als Gemische, z.B. als Racemate, vorliegen.

Die Verbindungen der Formel I kann man in an sich bekannter Weise dadurch herstellen, dass man

a) einen der Säure der Formel I entsprechenden $C_{1-4}$-Alkylester verseift oder

b) in einer Säure der Formel

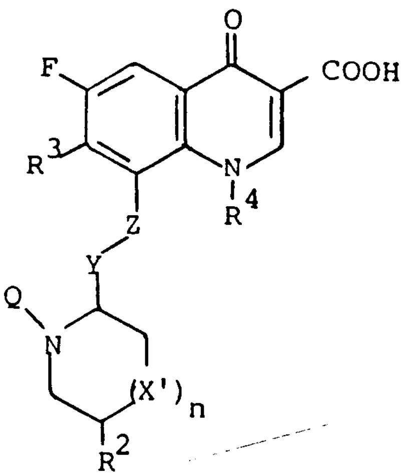

II

worin X' eine Gruppe N-R oder N-Q', Q und Q' Wasserstoff oder eine Stickstoffschutzgruppe, $R^3$ Halogen, und $R^4$ Wasserstoff sind oder $R^4$ die Bedeutung von $R^1$ hat, und $R^1$, $R^2$, R, Y, Z und n die gleiche Bedeutung wie in der Formel I haben,
allfällig vorhandene Stickstoffschutzgruppen abspaltet und das entstandene sekundäre Amin cyclisiert oder
c) eine Säure der Formel

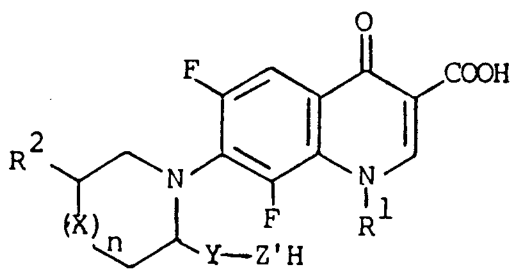

III

worin Z' Sauerstoff oder Schwefel ist und n, X, Y, $R^1$ und $R^2$ die gleiche Bedeutung wie in der Formel I haben,
cyclisiert,
d) gewünschtenfalls eine erhaltene Verbindung der Formel I mit einem die Gruppe R bzw. die Gruppe $R^1$ einführenden Mittel umsetzt,
e) gewünschtenfalls die Hydroxygruppe $R^2$ in einer erhaltenen Verbindung der Formel I in eine Aminogruppe $N(R^e,R^f)$ umwandelt,
f) gewünschtenfalls den Vinylrest $R^1$ in einer erhaltenen Verbindung der Formel I in einen Cyclopropylrest umwandelt,
g) gewünschtenfalls eine erhaltene Verbindung der Formel I in Form eines Salzes isoliert.

In der Verseifung a) wird zweckmässig der der Säure I entsprechende Aethylester mit einer Base, z.B. einer anorganischen Base, wie einem Alkali- oder Erdalkalimetallhydroxyd oder -carbonat, in einem Lösungsmittel, z.B. einem Niederalkanol, wie Aethanol, unter Erhitzen, z.B. bis auf Rückflusstemperatur des Reaktionsgemisches umgesetzt.

Als Stickstoffschutzgruppe Q bzw. Q' in den Ausgangssäuren II kommen z.B. Benzyl und p-Nitrobenzyl in Frage. Als Halogen $R^3$ sind Fluor und Chlor bevorzugt. Das Verfahren b) kann so durchgeführt werden, dass man zunächst allfällige Stickstoffschutzgruppen Q bzw. Q' hydrogenolytisch abspaltet und das entstandene sekundäre Amin zweckmässig in situ durch eine intramolekulare nucleophile Substitutionsreaktion cyclisiert. Die hydrogenolytische Abspaltung von Schutzgruppen kann in Gegenwart eines Hydrierungskatalysators, wie Palladium auf Kohle (Pd/C), in saurem Medium, z.B. in Eisessig, durchgeführt werden. Falls $R^3$ Fluor ist, kann man die darauffolgende Cyclisierung in einem Lösungsmittel, wie Acetonitril, bei einer Temperatur bis zur Rückflusstemperatur, z.B. bei 50-60 °C, durchführen. Falls $R^3$ Chlor ist, arbeitet man zweckmässig in einem Lösungsmittel, wie Pyridin bei einer Temperatur bis zur Rückflusstemperatur, z.B. bei 50-80 °C.

Die Cyclisierung c) kann man in einem Lösungsmittel, wie Dimethylformamid (DMF) oder Dimethylsulfoxyd (DMSO), in Gegenwart einer Base, wie Natriumhydrid, unter Erhitzen bei einer Temperatur bis zur Rückflusstemperatur, z.B. bei 130-140 °C, durchführen.

Als die Gruppe R bzw. $R^1$ einführende Mittel können Halogenide, wie Jodide, z.B. Aethyljodid; Mesylate oder Tosylate verwendet werden. Die Reaktion d) kann man, gegebenenfalls unter intermediärem Schutz eines der beiden zu substituierenden N-Atome, z.B. mit einer der weiter oben genannten Stickstoffschutzgruppen, in einem Lösungsmittel, wie DMF, in Gegenwart einer Base, z.B. einer anorganischen Base, wie einem Alkali- oder Erdalkalimetallhydroxyd oder -carbonat, beispielsweise Kaliumcarbonat, unter Erhitzen, z.B. auf 80 °C, durchführen.

Die Umwandlung e) kann man dadurch bewerkstelligen, dass man zunächst einen Alkohol I, worin $R^2$ Hydroxy ist, verestert, z.B. mit p-Tosylchlorid oder Mesylchlorid, in einem Lösungsmittel, wie Methylenchlorid, Dioxan oder Tetrahydrofuran (THF) in Gegenwart einer Base, wie Triäthylamin, bei Raumtemperatur, und das erhaltene Tosylat oder Mesylat mit einem Ueberschuss eines Amins $HN(R^e,R^f)$ in einem Lösungsmittel, wie THF, Dioxan oder DMSO, bei einer Temperatur bis zu etwa 100 °C umsetzt. Alternativ kann man den Alkohol I zum entsprechenden Keton oxydieren, z.B. mittels Jones-Reagens ($CrO_3/H_2SO_4$) in Aceton, bei 20-35 °C, und das Keton zunächst mit dem Amin $HN(R^e,R^f)$, gegebenenfalls in Gegenwart eines Molekularsiebs, in einem Lösungsmittel, wie THF oder Dioxan, bei Raumtemperatur umsetzen und dann das erhaltene Produkt reduzieren, z.B. mit Natriumborhydrid in Aethanol bei 20-60 °C oder mit Ameisensäure nach Leuckart-Wallach (Org. Reactions 5, 1949, 301). Das dem Alkohol I entsprechende Keton kann auch mit Phenylhydrazon in einem Lösungsmittel, wie einem Alkohol, z.B. Aethanol, bei Raumtemperatur in das entsprechende Phenylhydrazid übergeführt werden. Letzteres kann man dann mit einem Reduktionsmittel, wie Natriumamalgam, im besagten Lösungsmittel, unter Erhitzen bis auf Rückflusstemperatur in das Amin der Formel I, worin $R^2$ Amino ist, umwandeln.

Die Umwandlung f) kann man nach der Methode von Simmons-Smith mittels Jodmethylzinkjodid oder durch Reaktion des Enamins der Formel I mit Diazomethan in Gegenwart von Kupfer(I)-chlorid bewerkstelligen.

Die in der Verfahrensvariante a) verwendeten $C_{1-4}$-Alkylester kann man aus den den Säuren II entsprechenden $C_{1-4}$-Alkylestern herstellen, z.B. wie weiter oben für die Verfahrensvariante b) beschrieben. Solche an einem (oder an beiden) N-Atom(en) unsubstituierte Ester können gegebenenfalls mit einem die Gruppe R (bzw. $R^1$) einführenden Mittel umgesetzt werden, z.B. wie weiter oben für die Verfahrensvariante d) beschrieben.

Die Säuren II und III kann man durch Verseifung der entsprechenden $C_{1-4}$-Alkylester herstellen, z.B. in Analogie zur Verfahrensvariante a). Die besagten Ausgangssäuren und -ester sind neu. Sie können in an sich bekannter Weise, insbesondere auf die im folgenden beschriebenen Weise oder in Analogie dazu, hergestellt werden.

So kann man die Säuren der Formel II, worin $R^4$ Wasserstoff ist, ausgehend von den Verbindungen der Formel

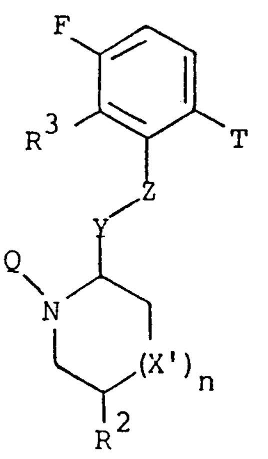

IV

worin T Nitro, Amino oder 2-Bis(carbo-$C_{1-4}$-alkoxy)vinylamino ist, und n, Q, $R^2$, $R^3$, X', Y und Z die obige Bedeutung haben,
herstellen. Die Reduktion der Nitro- zur Aminogruppe T kann z.B. mit Zinkstaub in Essigsäure durchgeführt werden. Man kann ein erhaltenes Amin IV durch Erhitzen, z.B. auf 110 °C mit einem $C_{1-4}$-Alkoxymethylenmalonsäuredi-$C_{1-4}$-alkylester, z.B. mit Aethoxymethylenmalonsäurediäthylester, in das entsprechende Vinylamin IV überführen. Letzteres kann, z.B. mit Aethylpolyphosphat unter inerter Atmosphäre und erhöhter Temperatur, beispielsweise unter $N_2$ bei 100 °C, zu einem der Säure der Formel II, worin $R^4$ Wasserstoff ist, entsprechenden $C_{1-4}$-Alkylester cyclisiert werden.

Die Verbindungen IV mit T = Nitro können in an sich bekannter Weise hergestellt werden. So kann man eine Verbindung IV mit Z = Sauerstoff oder Schwefel durch Umsetzung eines Nitrobenzolderivats V mit einem Piperazin oder Pyrrolidinderivat VI,

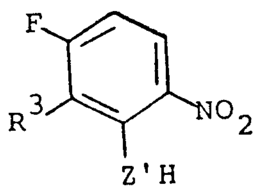

V

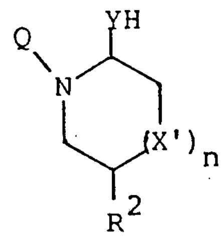

VI

worin n, Z' Q, $R^2$, $R^3$, X' und Y die obige Bedeutung haben,
z.B. in Gegenwart von Triphenylphosphin und Diazadicarbonsäurediäthylester, in einem Lösungsmittel, wie THF, herstellen.

Wie bereits erwähnt, kann man die Säuren III durch Verseifung der entsprechenden $C_{1-4}$-Alkylester herstellen. Letztere können durch nucleophile Substitution eines Esters der Formel VII mit einem Amin der Formel VIII

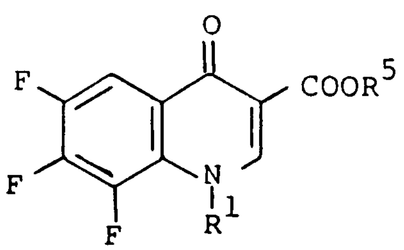

VII

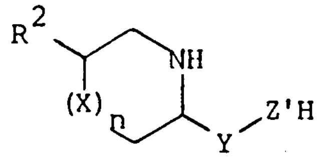

VIII

worin $R^5$ $C_{1-4}$-Alkyl ist und n, $R^1$, $R^2$, Y und Z' die obige Bedeutung haben,
in einem Lösungsmittel, wie N-Methylpyrrolidin oder α-Picolin, unter Erhitzen, z.B. auf 80 °C, hergestellt werden.

Die trifluorierten Chinolinderivate VII kann man durch Umsetzung der tetrafluorierten Benzolderivaten der Formel

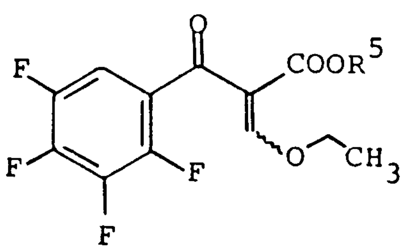

IX

mit eihem Amin $R^1$-$NH_2$, in einem Lösungsmittel, wie Methylenchlorid, Aethanol oder Chloroform, herstellen. Falls $R^1$ eine primäre oder sekundäre Aminogruppe ist, muss man deren N-Atom intermediär, z.B. mit einer Dimethyl-t-butylsilylgruppe, schützen.

Die Verbindungen der Formeln V bis IX sind bekannt oder können in an sich bekannter Weise hergestellt werden.

Unter den Verbindungen der Formeln I und II sind diejenigen bevorzugt, worin n die Zahl 1, R Wasserstoff, $C_{1-4}$-Alkyl oder $C_{2-4}$-Alkylen-N($R^a$,$R^b$), und $R^2$ Wasserstoff ist. Unter den Verbindungen der Formeln I, II und III sind diejenigen bevorzugt, worin R Wasserstoff, $C_{1-4}$-Alkyl, insbesondere Methyl oder Aethyl, oder ringsubstituiertes Benzyl, insbesondere p-Nitrobenzyl; und/oder worin Y Methylen und Z bzw. Z' Sauerstoff; und/oder worin $R^1$ bzw. $R^4$ Wasserstoff, gegebenenfalls fluoriertes $C_{1-4}$-alkyl, insbesondere

Methyl, Aethyl oder 2-Fluoräthyl, $C_{3-6}$-Cycloalkyl, insbesondere Cyclopropyl; $N(R^c,R^d)$, insbesondere Methylamino, oder substituiertes Phenyl, insbesondere p-Fluorphenyl, ist. Besonders bevorzugt sind die Verbindungen der Formeln I, II und III, worin $R^2$ Wasserstoff oder Hydroxy; und/oder worin R Wasserstoff oder Methyl; und/oder worin $R^1$ bzw. $R^4$ Aethyl, Cyclopropyl oder Methylamino ist.

Beispiele von bevorzugten Verbindungen sind

1-Cyclopropyl-6,8-difluor-1,4-dihydro-7-(2-hydroxymethyl-4-methyl-1-piperazinyl)-4-oxochinolin-3-carbonsäure

1-Aethyl-6-fluor-1,4,8,9,10,11,11a,12-octahydro-10-methyl-4-oxopyrazino[1',2':4,5][1,4]oxazino[3,2-h]-chinolin-3-carbonsäure

1-Aethyl-6-fluor-1,4,8,9,10,11,11a,12-octahydro-4-oxopyrazino[1',2':4,5][1,4]oxazino[3,2-h]chinolin-3-carbonsäure.

Weitere Beispiele von Verbindungen der Formeln I bzw. III sind

1-Cyclopropyl-6-fluor-1,4,8,9,10,11,11a,12-octahydro-4-oxopyrazino[1',2':4,5][1,4]-oxazino[3,2-h]chinolin-3-carbonsäure

1-Cyclopropyl-6-fluor-10-methyl-1,4,8,9,10,11,11a,12-octahydro-4-oxopyrazino[1',2':4,5][1,4]oxazino[3,2-h]chinolin-3-carbonsäure

6-Fluor-1-(p-fluorphenyl)-1,4,8,9,10,11,11a,12-octahydro-4-oxopyrazino[1',2':4,5][1,4]oxazino[3,2-h]-chinolin-3-carbonsäure

6-Fluor-1-(p-fluorphenyl)-10-methyl-1,4,8,9,10,11, 11a,12-octahydro-4-oxopyrazino[1',2':4,5][1,4]oxazino-[3,2-h] chinolin-3-carbonsäure

6-Fluor-1-(2-fluoräthyl)-1,4,8,9,10,11,11a,12-octahydro-4-oxopyrazino[1',2':4,5][1,4]oxazino[3,2-h]chinolin-3-carbonsäure

6-Fluor-1-(2-fluoräthyl)-10-methyl-1,4,8,9,10,11,11a,12-octahydro-4-oxopyrazino[1',2':4,5][1,4]oxazino-[3,2-h]chinolin-3-carbonsäure

6-Fluor-1-methylamino-1,4,8,9,10,11,11a,12-octahydro-4-oxopyrazino[1',2':4,5][1,4]oxazino[3,2-h]-chinolin-3-carbonsäure

6-Fluor-10-methyl-1-methylamino-1,4,8,9,10,11,11a,12-octahydro-4-oxopyrazino[1',2':4,5][1,4]oxazino-[3,2-h]chinolin-3-carbonsäure

(S)-4-Aethyl-11-fluor-1,4,6a,7,8,9-hexahydro-1-oxo-6H-pyrrolo[1',2':4,5][1,4]oxazino[3,2-h]chinolin-2-carbonsäure

6a(S),8(S)-4-Aethyl-11-fluor-1,4,6a,7,8,9-hexahydro-8-hydroxy-1-oxo-6H-pyrrolo[1',2':4,5][1,4]oxazino[3,2-h]chino lin-2-carbonsäure

6a(S)-8-Amino-4-äthyl-11-fluor-1,4,6a,7,8,9a-hexahydro-1-oxo-6H-pyrrolo[1',2':4,5][1,4]oxazino[3,2-h]-chinolin-2-carbonsäure

6a(S)-8-Amino-4-cyclopropyl-11-fluor-1,4,6a,7,8,9-hexahydro-1-oxo-6H-pyrrolo[1',2':4,5][1,4]oxazino[3,2-h] chinolin-2-carbonsäure

6a(S),8(S)-11-Fluor-4-(p-fluorphenyl)-1,4,6a,7,8,9-hexahydro-8-hydroxy-1-oxo-6H-pyrrolo[1',2':4,5]-oxazino[3,2-h] chinolin-2-carbonsäure

6a(S)-8-Amino-11-fluor-4-(p-fluorphenyl)-1,4,6a,7,8,9-hexahydro-1-oxo-6H-pyrrolo[1',2':4,5][1,4]oxazino-[3,2-h]chinolin-2-carbonsäure

6a(S),8(S)-11-Fluor-1,4,6a,7,8,9-hexahydro-8-hydroxy-4-methylamino-1-oxo-6H-pyrrolo[1',2':4,5][1,4]-oxazino[3,2-h] chinolin-2-carbonsäure

6a(S)-8-Amino-11-fluor-1,4,6a,7,8,9-hexahydro-4-methylamino-1-oxo-6H-pyrrolo[1',2':4,5][1,4]oxazino-[3,2-h] chinolin-2-carbonsäure

1-Aethyl-6,8-difluor-1,4-dihydro-7-[2-hydroxymethyl-1-piperazinyl]-4-oxochinolin-3-carbonsäure

1-Aethyl-6,8-difluor-1,4-dihydro-7-[2-hydroxymethyl-4-methyl-1-piperazinyl]-4-oxochinolin-3-carbonsäure

1-Cyclopropyl-6,8-difluor-1,4-dihydro-7-[2-hydroxymethyl-1-piperazinyl]-4-oxochinolin-3-carbonsäure

6,8-Difluor-1,4-dihydro-1-(p-fluorphenyl)-7-[2-hydroxymethyl-1-piperazinyl]-4-oxochinolin-3-carbonsäure

6,8-Difluor-1,4-dihydro-1-(2-fluoräthyl)-7-[2-hydroxymethyl-1-piperazinyl]-4-oxochinolin-3-carbonsäure

6,8-Difluor-1,4-dihydro-1-methylamino-7-[2-hydroxymethyl-1-piperazinyl]-4-oxochinolin-3-carbonsäure.

Die erfindungsgemässen Chinolinderivate I und III sind pharmakologisch aktiv. Sie weisen antibakterielle Aktivität auf, wobei sie durch geringe akute Toxizitäten gekennzeichnet sind. Sie sind z.B. gegen grampositive aerobe Bakterien, wie S. aureus, S. pyogenes, S. faecalis oder S. pneumoniae, gegen Enterobacteriaceae, wie E. coli oder E. cloacae, gegen P. aeruginosa und A. anitratus wirksam. So wurden gegen einige dieser Erreger die nachstehenden minimal hemmenden Konzentrationen in µg/ml der folgenden Verbindungen ermittelt:

Verbindung A (Beispiel 1):

1-Aethyl-6-fluor-1,4,8,9,10,11,11a,12-octahydro-4-oxo -pyrazino[1',2':4,5][1,4]oxazino[3,2-h]chinolin-3-carbonsäure
Verbindung B (Beispiel 8a):
1-Cyclopropyl-6,8-difluor-1,4-dihydro-7-(2-hydroxymethyl-4-methyl-1-piperazinyl)-4-oxochinolin-3-carbonsäure
Verbindung C (Beispiel 2A):
1-Aethyl-6-fluor-1,4,8,9,10,11,11a,12-octahydro-10-methyl-4-oxopyrazino[1',2':4,5][1,4]oxazino[3,2-h]chinolin-3-carbonsäure
Verbindung D (Beispiel 8c):
6,8-Difluor-1,4-dihydro-1-(p-fluorphenyl)-7-(2-hydroxymethyl-4-methyl-1-piperazinyl)-4-oxochinolin-3-carbonsäure
Verbindung E (Beispiel 4A):
(S)-4-Cyclopropyl-11-fluor-1,4,6a,7,8,9 -hexahydro-1-oxo-6H-pyrrolo[1',2':4,5][1,4]oxazino[3,2-h]chinolin-2-carbonsäure
Verbindung F (Beispiel 3a):
1,10-Diäthyl-6-fluor-1,4,8,9,10,11,11a,12-octahydro-4-oxopyrazino[1',2':4,5][1,4]oxazino[3,2-h]chinolin-3-carbonsäure.

| | A | B | C | D | E | F |
|---|---|---|---|---|---|---|
| E. coli 1346 | <0,12 | 0,06 | 0,25 | 0,5 | 1 | 0,5 |
| E. coli 25922 | 0,25 | 0,06 | 0,25 | 0,25 | 0,5 | 0,5 |
| E. coli 1527E | <0,12 | 0,03 | <0,03 | 0,03 | 0,03 | 0,12 |
| E. cloacae P99 | <0,12 | 0,06 | 0,12 | 0,25 | 0,25 | 0,5 |
| S. aureus 25923 | | 0,5 | 1 | 1 | 0,12 | 2 |
| " 887 | 1 | 0,5 | 1 | 1 | 0,12 | 2 |
| " 743 | | 0,5 | 1 | 1 | 0,12 | 2 |
| S. faecalis 6 | 8 | 2 | 32 | 4 | 2 | 16 |
| S. pyogenes ß15 | 2 | 2 | 32 | 4 | 1 | 32 |
| S. pneumoniae BA | 2 | 2 | 32 | 2 | 1 | 16 |
| A. anitratus 5I-156 | 1 | 0,25 | 0,25 | 1 | 2 | 1 |

Die erfindungsgemässen Chinolinderivate können bei der Therapie und Prophylaxe von Krankheiten, insbesondere von bakteriellen Infektionen in Form pharmazeutischer Präparate mit direkter oder verzögerter Freigabe des Wirkstoffes in Mischung mit einem für die orale, rektale oder parenterale Applikation geeigneten organischen oder anorganischen inerten Trägermaterial, z.B. Wasser, Gelatine, Gummi arabicum, Milchzucker, Stärke, Magnesiumstearat, Talk, pflanzlichen Oelen, Polyalkylenglykolen und Vaseline, verwendet werden. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten weitere Hilfsstoffe, wie Konservierungs-, Stabilisierungs-, Netz- oder Emulgiermittel, Mittel zur geschmacklichen Verbesserung, Salze zur Veränderung des osmotischen Druckes oder Puffersubstanzen.

Die Herstellung der pharmazeutischen Präparate kann in dem Fachmann geläufiger Weise, nämlich dadurch erfolgen, dass man die Wirksubstanz mit dem zur therapeutischen Verabreichung geeigneten, nicht-toxischen, inerten Trägermaterial vermischt und das erhaltene Gemisch in die geeignete galenische Form bringt.

Als Dosierungsrichtlinie kann für eine Verbindung der Formel I oder III eine Menge von 10 $\mu$g bis 100 mg/kg, vorzugsweise etwa 4 mg/kg Körpergewicht pro Tag gelten.

Beispiel 1

1A) Eine Lösung von 0,165 g (0,44 mM) 1-Aethyl-6-fluor-1,4,8,9,10,11,11a,12-octahydro-4-oxo -pyrazino-[1',2':4,5][1,4]-oxazino[3,2-h]chinolin-3-carbonsäureäthylester in 10 ml Aethanol und 7,5 ml 0,1N NaOH wurde 90 Minuten bei 70°C gerührt. Die Lösung wurde mit 7,5 ml 0,1N HCl neutralisiert. Aethanol wurde unter vermindertem Druck entfernt und das feste Produkt wurde abfiltriert, gewaschen und getrocknet. Man erhielt 120 mg 1-Aethyl-6-fluor-1,4,8,9, 10,11,11a,12-octahydro-4-oxopyrazino[1',2':4,5][1,4]oxazino-[3,2-h]chinolin-3-carbonsäure, Smp. 266-268°C.

1B) Den Ausgangsester kann man wie folgt herstellen:

1Ba) Zu einer Lösung von 3,9 g (22,5 mM) 2,3-Difluor-5-nitrophenol, 7.1 g (27 mM) Triphenylphosphin und 8 g (0,27 mM) 1,4-Dibenzyl-2-hydroxymethylpiperazin in 250 ml THF wurde unter Rühren eine Lösung von 4.7 g (27 mM) Azadicarbonsäurediäthylester in 15 ml THF bei 15°C zugetropft. Dann wurde die gelbe Reaktionslösung noch 3 Stunden bei Raumtemperatur weitergerührt. Nach Einengung der Reaktionslösung wurde der ölige Rückstand in mehreren Portionen insgesamt mit 2 l heissem n-Hexan verrührt. Die Hexanlösung wurde abgetrennt und abgekühlt. Das ausgefallene Triphenylphosphinoxid wurde abgenutscht, das Filtrat wurde eingeengt. Chromatographie an Kieselgel mit Aether/n-Hexan (1:1) als Eluens ergab 7,4 g 1,4-Dibenzyl-2-[(6-nitro-2,3-difluorphenoxy)methyl]piperazin als braunes Oel.

1Bb) Zu einer Lösung von 7,1 g (15,7 mM) des Produkts von 1Ba) in 108 ml Eisessig und 36 ml Wasser wurden unter Rühren bei Raumtemperatur in Portionen 19 g (0,29 g Atom) Zinkstaub eingetragen. Die Temperatur stieg auf 40°C an. Dann wurde 1 Stunde bei 50°C gerührt und die warme Suspension wurde abgenutscht. Das abgekühlte Filtrat wurde mit 300 ml Aether versetzt. Der weisse Niederschlag wurde abgetrennt und verworfen und die Lösung wurde zur Trockene eingedampft. Der ölige Rückstand wurde in Wasser aufgenommen, mit konz. NaOH auf pH 5 gestellt und mit Aether extrahiert. Die Aetherphasen wurden nacheinander mit 5% $NaHCO_3$ und Wasser gewaschen, getrocknet und eingeengt. Das zurückgebliebene Oel wurde an Kieselgel mit Aether/n-Hexan (1:1) gereinigt und ergab 6,0 g 1,4-Dibenzyl-2-[(6-amino-2,3-difluorphenoxy)methyl]piperazin als bräunliches Oel.

1Bc) Aus einem Gemisch von 4,3 g (10,2 mM) des Produkts von 1Bb) und 2,4 g (11.2 mM) Aethoxymethylenmalonsäurediäthylester wurde während 40 Minuten bei 110°C Badtemperatur Aethanol abdestilliert. Das Reaktionsgemisch wurde abgekühlt. Umkristallisation aus Aethanol ergab 5,0 g [2-[(1,4-Dibenzyl-2-piperazinyl)methoxy] -3,4-difluoranilino]methylenmalonsäurediäthylester, Smp. 109-110°C.

1Bd) Ein Gemisch von 4.4 g (7,41 mM) des Produktes von 1Bc) und 30 g Aethylpolyphosphat wurde 10 Stunden unter $N_2$-Begasung bei 100°C gerührt. Das Gemisch wurde mit Eiswasser unter Kühlung vermischt und mit 4N NaOH auf pH 8 gestellt. Das feste Produkt wurde abgenutscht und aus Essigester/n-Hexan kristallisiert. Man erhielt 8-[(1,4-Dibenzyl-2-piperazinyl)methoxy]-6,7-difluor -1,4-dihydro-4-oxochinolin-3-carbonsäure, Smp. 158-159°C.

1Be) Eine Suspension von 1,38 g (2,4 mM) des Produktes von 1Bd), 0,85 g $K_2CO_3$ und 1,94 g (12 mM) Aethyljodid in 14 ml DMF wurde 2,5 Stunden bei 80°C gerührt. Die anorganischen Salze wurden abgenutscht, das Filtrat wurde eingeengt und der Eindampfrückstand wurde mit Aether verrührt. Die Lösung wurde eingeengt und der Rückstand wurde an Silicagel mit Essigester/n-Hexan (3:1) chromatographiert. Kristallisation aus Aether-Petroläther ergab 8-[(1,4-Dibenzyl-2-piperazinyl)methoxy]-6,7-difluor -1-äthyl-1,4-dihydro-4-oxochinolin-3-carbonsäureäthylester, Smp. 121-122°C.

1Bf) Eine Suspension von 0,5 g (0,86 mM) des Produktes von 1Be) und 0,5 g 5% Pd/C in 10 ml Eisessig wurde unter 10 bar Wasserstoffdruck bei 25°C während 240 Minuten hydriert. Der Katalysator wurde abgenutscht und mit 10 ml Eisessig nachgewaschen. Dann wurde das Lösungsmittel bei 25°C unter 0,4 mBa entfernt. Der Rückstand wurde in 10 ml Acetonitril aufgenommen und 45 Minuten am Rückfluss gekocht. Die entstandene Suspension wurde auf 0°C abgekühlt und abgenutscht. Das feste Produkt wurde in 2 ml Wasser gelöst und die Lösung wurde mit 0,1N NaOH auf pH 8 gestellt. Das ausgefallene Produkt wurde abgenutscht, gewaschen und getrocknet. Man erhielt 1-Aethyl-6-fluor-1,4,8,9,10,11,11a,12-octahydro-4-oxopyrazino[1',2':4,5][1,4]oxazino[3,2-h]chinolin-3-carbon säureäthylester, Smp. 266-268°C.

Beispiel 2

2A) Eine Lösung von 0,24 g (0,61 mM) 1-Aethyl-6-fluor-1,4,8,9,10,11,11a,12-octahydro-10-methyl-4-oxopyrazino[1',2':4,5][1,4]oxazino[3,2-h]chinolin-3-carbonsäureäthylester in 15 ml Aethanol und 6,8 ml 0,1N NaOH wurde 1 Stunde bei 85°C gerührt. Die Reaktionslösung wurde mit 6,8 ml 0.1N HCl neutralisiert und eingedampft, und das feste Produkt abgenutscht. Kristallisation aus Aethanol ergab 1-Aethyl-6-fluor-1,4,8,9,10,11,11a,12-octahydro-10-methyl-4-oxopyrazino[1',2':4,5][1,4]oxazino[3,2-h]-chinolin-3-carbonsäure, Smp. >270°C.

2B) Der Ausgangsester kann man wie folgt herstellen:

Zu einer Lösung von 0,6 g (16 mM) 1-Aethyl-6-fluor-1,4,8,9,10,11,11a,12-octahydro-4-oxopyrazino-[1',2':4,5] [1,4]oxazino[3,2-h]chinolin-3-carbonsäureäthylester (Beispiel 1Bf) in 40 ml Aceton wurden 0,33 g (24 mM) wasserfreies Kaliumcarbonat und 0,34 g (24 mM) Methyljodid zugegeben. Die Suspension wurde 90 Minuten bei Raumtemperatur gerührt. Das Reaktionsgemisch wurde filtriert und das Filtrat eingeengt. Der Rückstand wurde bereinigt und ergab nach Umkristallisation 1-Aethyl-6-fluor-1,4,8,9,10,11, 11a,12-octahydro-10-methyl-4-oxopyrazino[1',2':4,5][1,4] oxazino[3,2-h]chinolin-3-carbonsäureäthylester, Smp. 191-192 °C.

Beispiel 3

Analog Beispiel 2 erhält man

3a) aus 1,10-Diäthyl-6-fluor-1,4,8,9,10,11,11a,12-octahydro-4-oxopyrazino[1',2':4,5][1,4]oxazino[3,2-h]-chinolin-3-carbonsäureäthylester, Smp. 184-186 °C

die 1,10-Diäthyl-6-fluor-1,4,8,9,10,11,11a,12-octahydro-4-oxopyrazino[1',2':4,5][1,4]oxazino[3,2-h]-chinolin-3-carbonsäure, Smp. 261-264 °C

3b) aus 1-Aethyl-6-fluor-1,4,8,9,10,11,11a,12-octahydro-10-(p-nitrobenzyl)-4-oxopyrazino[1',2':4,5][1,4]-oxazino [3,2-h]chinolin-3-carbonsäureäthylester, Smp. 182-183 °C

die 1-Aethyl-6-fluor-1,4,8,9,10,11,11a,12-octahydro-10-(p-nitrobenzyl)-4-oxopyrazino[1',2':4,5]oxazino-[3,2-h] chinolin-3-carbonsäure, Smp. 240-243 °C.

Beispiel 4

4A) Zu einer Lösung von 0,364 g (1 mM) 1-Cyclopropyl-6,8-difluor-1,4-dihydro-7-[(S)-2-hydroxymethyl-1-pyrrolidinyl]-4-oxochinolin-3-carbonsäure in 8 ml DMF wurden 9,2 mg 55% NaH-Dispersion gegeben. Nach beendeter $H_2$-Entwicklung wurde 35 Minuten bei 140 °C gerührt, das Lösungsmittel wurde abgedampft, der Rückstand wurde in Wasser aufgenommen, mit Eisessig auf pH 6 gestellt und abgenutscht. Kristallisation aus Aethanol ergab (S)-4-Cyclopropyl-11-fluor-1,4,6a, 7,8,9-hexahydro-1-oxo-6H-pyrrolo[1',2':4,5][1,4]oxazino [3,2-h]chinolin-2-carbonsäure, Smp. >275 °C (Zers.), MS: 344 ($M^+$, 58%), 300 (100), 271 (12), 245 (8), 216 (17), 189 (4), 158 (4), 41 (24).

4B) Das Ausgangsmaterial kann man wie folgt herstellen:

Eine Lösung von 3.1 g (0,01 mM) 1-Cyclopropyl-1,4-dihydro-4-oxo-6,7,8-trifluorchinolin-3-carbonsäureäthylester und 3,03 g (0,03 mM) L-Prolinol in 15 ml N-Methylpyrrolidin wurden 2,5 Stunden bei 80 °C gerührt. Das Lösungsmittel wurde unter vermindertem Druck abdestilliert und der ölige Rückstand wurde aus Essigester/Aether kristallisiert. Analog Beispiel 1A wurde der entstandene Ester verseift. Man erhielt 1-Cyclopropyl-6,8-difluor-1,4-dihydro-7-[(S)-2-hydroxymethyl-1-pyrrolidinyl]-4-oxochinolin-3-carbonsäure, Smp. 209-210 °C.

Beispiel 5

Analog Beispiel 4 erhielt man unter Verwendung von 3-Hydroxy-L-prolinol an Stelle von L-Prolinol über die 1-Cyclopropyl-6,8-difluor-1,4-dihydro-7-[2(S),4(S)-4-hydroxy-2-hydroxymethyl-1-pyrrolidinyl]-4-oxochinolin-3-carbonsäure, Smp. >250 °C (Zersetzung)

die 6a(S),8(S)-4-Cyclopropyl-11-fluor-1,4,6a,7,8,9-hexahydro-8-hydroxy-1-oxo-6H-pyrrolo[1',2':4,5][1,4]-oxazino[3,2-h]chinolin-2-carbonsäure, Smp. >270 °C (Zersetzung), MS: 360 ($M^+$, 30%), 316 (100), 267 (13), 247 (8), 245 (8), 217 (8). 101 (13).

Beispiel 6

Analog Beispiel 4 erhielt man

6a) aus 1,4-Dihydro-1-(p-fluorphenyl)-4-oxo-6,7,8-trifluorchinolin-3-carbonsäureäthylester, über 6,8-Difluor-1,4-dihydro-1-(p-fluorphenyl)-7-[(S)-2-hydroxymethyl-1-pyrrolidinyl]-4-oxochinolin-3-carbonsäure, Smp. 275-277 °C (Zersetzung)

die (S)-11-Fluor-4-(p-fluorphenyl)-1,4,6a,7,8,9-hexahydro-1-oxo-6H-pyrrolo[1',2':4,5][1,4]oxazino[3,2-h]chinolin-2-carbonsäure, Smp. >280 °C (Zersetzung), MS: 398 ($M^+$, 26%), 354 (100), 332 (10), 313 (6), 299 (6), 245 (20), 95 (12)

6b) aus 1,4-Dihydro-1-methylamino-4-oxo-6,7,8-trifluorchinolin-3-carbonsäureäthylester über 6,8-Difluor-1,4-dihydro-7-[(S)-2-hydroxymethyl-1-pyrrolidinyl]-1-methylamino-4-oxochinolin-3-carbonsäure, Smp.

179-181 °C

die (S)-11-Fluor-1,4,6a,7,8,9-hexahydro-4-methylamino-1-oxo-6H-pyrrolo[1',2':4,5][1,4]oxazino[3,2-h]-chinolin-2-carbonsäure, MS: 333 ($M^+$, 72%), 289 (100), 272 (64), 260 (98), 234 (18), 231 (46), 205 (12), 191 (12), 135 (9), 41 (44)

6c) aus 1,4-Dihydro-1-(2-fluoräthyl)-4-oxo-6,7,8-trifluorchinolin-3-carbonsäureäthylester über 6,8-Difluor-1,4-dihydro-1-(2-fluoräthyl)-7-[(S)-2-hydroxymethyl-1-pyrrolidinyl]-4-oxochinolin-3-carbonsäure, Smp. 195-197 °C

die (S)-11-Fluor-4-(2-fluoräthyl)-1,4,6a,7,8,9-hexahydro-1-oxo-6H-pyrrolo[1',2':4,5][1,4]oxazino[3,2-h)-chinolin-2-carbonsäure, MS: 350 ($M^+$, 42%), 306 (100), 286 (18), 273 (18), 245 (10), 153 (12), 41 (21).

Beispiel 7

Analog Beispiel 1 erhielt man aus 8-[(1,4-Dibenzyl-2-piperazinyl)methoxy]-6,7-difluor-1,4-dihydro-4-oxochinolin-3-carbonsäure (Beispiel 1Bd) unter Verwendung von Methyljodid anstatt Aethyljodid (Beispiel 1Be)

die 6-Fluor-1,4,8,9,10,11,11a,12-octahydro-1-methyl-4-oxopyrazino[1',2':4,5][1,4]oxazino[3,2-h]chinolin-3-carbonsäure, Smp. 236-240 °C (Zersetzung).

Beispiel 8

Analog Beispiel 4B unter Verwendung von 4-Methyl-2-piperazinylmethanol anstelle von L-Prolinol wurden folgende Verbindungen erhalten:

a) 1-Cyclopropyl-6,8-difluor-1,4-dihydro-7-[2-hydroxymethyl-4-methyl-1-piperazinyl]-4-oxochinolin-3-carbonsäure, Smp. 213-214 °C

b) 6,8-Difluor-1,4-dihydro-1-(2-fluoräthyl)-7-[2-hydroxymethyl-4-methyl-1-piperazinyl]-4-oxochinolin-3-carbonsäure, Smp. des Dihydrochlorids >260 °C (Zersetzung)

c) 6,8-Difluor-1,4-dihydro-1-(p-fluorphenyl)-7-[2-hydroxymethyl-4-methyl-1-piperazinyl]-4-oxochinolin-3-carbonsäure, Smp. 265-267 °C

d) 6,8-Difluor-1,4-dihydro-7-[2-hydroxymethyl-4-methyl-1-piperazinyl]-1-methylamino-4-oxochinolin-3-carbonsäure, MS: 400 [$(M+H)^+$, 1%), 368 (76), 324 (24), 309 (10), 281 (16), 70 (100), 36 (40).

Beispiel 9

Es werden in üblicher Weise Tabletten folgender Zusammensetzung hergestellt:

| | | | |
|---|---|---|---|
| Wirkstoff | 100 | 200 | 400 mg |
| Pulverisierter Milchzucker | 25 | 70 | 200 |
| Mikrokristalline Cellulose | 70 | 70 | 100 |
| Maisstärke | 30 | 60 | 50 |
| Polyvinylpyrrolidon | 10 | 20 | 30 |
| Na-Carboxymethylstärke | 10 | 20 | 30 |
| Talk | 3 | 8 | 16 |
| Magnesiumstearat | 2 | 2 | 4 |
| | 250 | 450 | 830 mg |

**Patentansprüche**
**Patentansprüche für folgende Vertragsstaaten: BE, CH, DE, FR, GB, IT, LI, LU, NL, SE**

**1.** Chinolinderivate der Formel

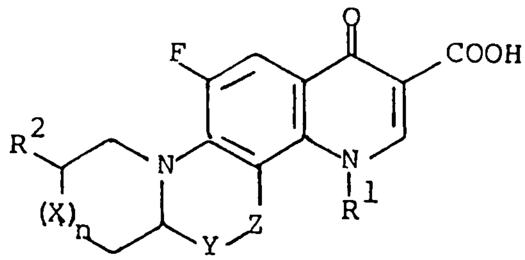

I

worin

| | |
|---|---|
| n | die Zahl 1 oder 0 |
| X | eine Gruppe N-R |
| R | Wasserstoff, $C_{1-4}$-Alkyl, $C_{2-4}$-Alkylen-N($R^a$,$R^b$) oder gegebenenfalls durch bis zu 3-Substituenten aus der Gruppe von Hydroxy, Halogen, $C_{1-4}$-Alkyl, $C_{1-4}$-Alkoxy oder Nitro ringsubstituiertes Benzyl |
| Y | Methylen oder Aethylen |
| Z | Methylen , 0 oder S |
| $R^1$ | $C_{3-6}$-Cycloalkyl, N($R^c$,$R^d$), gegebenenfalls durch bis zu 3 Substituenten aus der Gruppe von Hydroxy, Halogen, $C_{1-4}$-Alkyl, $C_{1-4}$-Alkoxy oder Nitro substituiertes Phenyl oder gegebenenfalls fluoriertes $C_{1-4}$-Alkyl oder $C_{2-4}$-Alkenyl |
| $R^2$ | Wasserstoff, $C_{1-4}$-Alkyl oder, falls n = 0 ist, auch OH oder N($R^e$,$R^f$) |
| $R^a$ bis R | $^g$ Wasserstoff oder $C_{1-4}$-Alkyl sind, oder N($R^a$,$R^b$) ein 5- oder 6-gliedriger gesättigter Rest, gegebenenfalls mit einem zusätzlichen Heteroatom 0 oder N-$R^g$ ist, |

und physiologisch verträgliche Salze davon.

**2.** Chinolinderivate der Formel

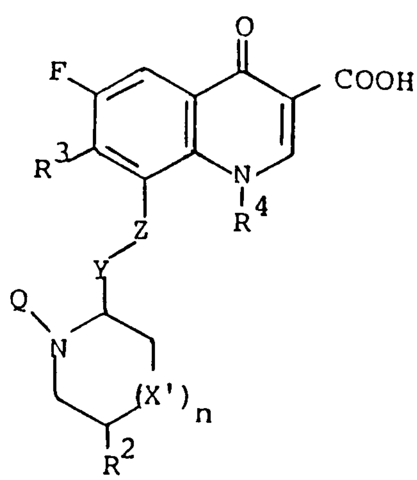

II

worin X' eine Gruppe N-R oder N-Q', Q und Q' Wasserstoff oder eine Stickstoffschutzgruppe. $R^3$ Halogen, und $R^4$ Wasserstoff sind oder $R^4$ die Bedeutung von $R^1$ hat, und $R^1$, $R^2$, R, Y, Z und n die gleiche Bedeutung wie in der Formel I haben.

**3.** Chinolinderivate der Formel

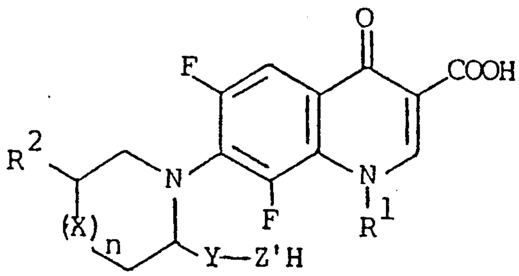

III

worin Z' Sauerstoff oder Schwefel ist und n, X, Y, $R^1$ und $R^2$ die gleiche Bedeutung wie in der Formel I haben.

**4.** Verbindungen nach Anspruch 1 oder 2, worin n die Zahl 1, R Wasserstoff, $C_{1-4}$-Alkyl oder $C_{2-4}$-Alkylen-N($R^a$,$R^b$), und $R^2$ Wasserstoff ist.

**5.** Verbindungen nach Anspruch 1, 2 oder 3, worin R Wasserstoff, $C_{1-4}$-Alkyl, insbesondere Methyl oder Aethyl, oder ringsubstituiertes Benzyl, insbesondere p-Nitrobenzyl, ist.

**6.** Verbindungen nach Anspruch 1, 2 oder 3, worin Y Methylen und Z bzw. Z' Sauerstoff sind.

**7.** Verbindungen nach Anspruch 1, 2 oder 3, worin $R^1$ bzw. $R^4$ Wasserstoff, gegebenenfalls fluoriertes $C_{1-4}$-Alkyl, insbesondere Methyl, Aethyl oder 2-Fluoräthyl, $C_{3-6}$-Cycloalkyl, insbesondere Cyclopropyl; N($R^c$,$R^d$), insbesondere Methylamino, oder substituiertes Phenyl, insbesondere p-Fluorphenyl, ist.

**8.** Verbindungen nach Anspruch 1, 2 oder 3, worin $R^2$ Wasserstoff oder Hydroxy ist.

**9.** Verbindungen nach Anspruch 5, worin R Wasserstoff oder Methyl ist.

**10.** Verbindungen nach Anspruch 7, worin $R^1$ bzw. $R^4$ Aethyl, Cyclopropyl oder Methylamino ist.

**11.** Verbindungen nach Anspruch 1, 9 oder 10 aus der Gruppe der folgenden

1-Cyclopropyl-6,8-difluor-1,4-dihydro-7-(2-hydroxymethyl-4-methyl-1-piperazinyl)-4-oxochinolin-3-carbonsäure

1-Aethyl-6-fluor-1,4,8,9,10,11,11a,12-octahydro-10-methyl-4-oxopyrazino[1',2':4,5][1,4]oxazino[3,2-h]chinolin-3-carbonsäure

1-Aethyl-6-fluor-1,4,8,9,10,11,11a,12-octahydro-4-oxo-pyrazino[1',2':4,5][1,4]oxazino[3,2-h]chinolin-3-carbonsäure.

**12.** Verbindungen nach einem der Ansprüche 1, 3 oder 11, zur Verwendung als therapeutischer, insbesondere antibakterieller Wirkstoff.

**13.** Verfahren zur Herstellung einer Verbindung nach Anspruch 1, dadurch gekennzeichnet, dass man

a) einen der Säure der Formel 1 entsprechenden $C_{1-4}$-Alkylester verseift oder

b) in einer Säure der Formel

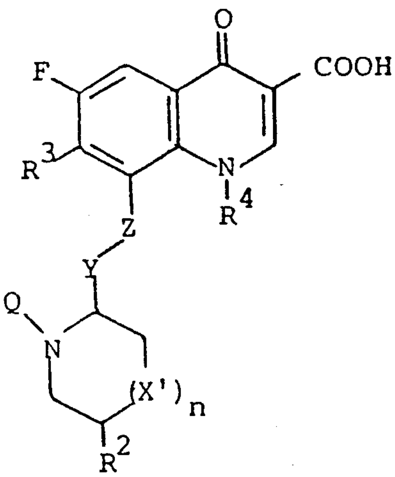

II

worin X' eine Gruppe N-R oder N-Q', Q und Q' Wasserstoff oder eine Stickstoffschutzgruppe, $R^3$ Halogen, und $R^4$ Wasserstoff sind oder $R^4$ die Bedeutung von $R^1$ hat, und $R^1$, $R^2$, R, Y, Z und n die gleiche Bedeutung wie in der Formel I haben,
allfällig vorhandene Stickstoffschutzgruppen abspaltet und das entstandene sekundäre Amin cyclisiert oder
c) eine Säure der Formel

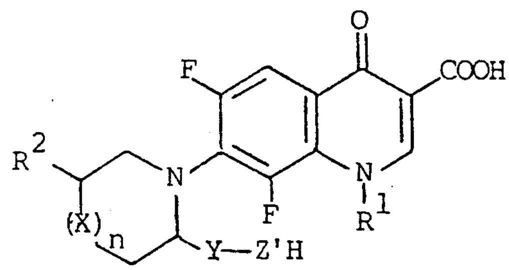

III

worin Z' Sauerstoff oder Schwefel ist und n, X, Y, $R^1$ und $R^2$ die gleiche Bedeutung wie in der Formel I haben,
cyclisiert,
d) gewünschtenfalls eine erhaltene Verbindung der Formel I mit einem die Gruppe R bzw. die Gruppe $R^1$ einführenden Mittel umsetzt,
e) gewünschtenfalls die Hydroxygruppe $R^2$ in einer erhaltenen Verbindung der Formel I in eine Aminogruppe $N(R^e,R^f)$ umwandelt,
f) gewünschtenfalls den Vinylrest $R^1$ in einer erhaltenen Verbindung der Formel I in einen Cyclopropylrest umwandelt,
g) gewünschtenfalls eine erhaltene Verbindung der Formel I in Form eines Salzes isoliert.

**14.** Verfahren zur Herstellung einer Verbindung nach Anspruch 3, dadurch gekennzeichnet, dass man einen der Säure der Formel III entsprechenden $C_{1-4}$-Alkylester verseift.

**15.** Präparat auf der Basis einer Verbindung nach einem der Ansprüche 1, 3 oder 11, insbesondere für Therapie und Prophylaxe von bakteriellen Infektionen.

**16.** Verwendung einer Verbindung nach einem der Ansprüche 1, 3 oder 11 zur Herstellung eines Präparats nach Anspruch 15.

**Patentansprüche für folgenden Vertragsstaat: AT**

**1.** Verfahren zur Herstellung von Chinolinderivaten der Formel

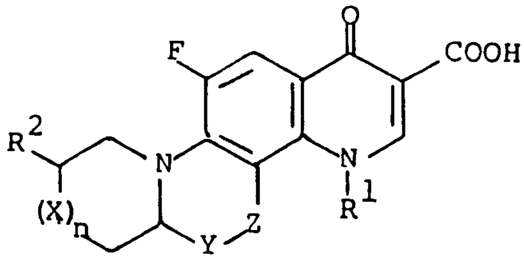

I

worin

| | |
|---|---|
| n | die Zahl 1 oder 0 |
| X | eine Gruppe N-R |
| R | Wasserstoff, $C_{1-4}$Alkyl, $C_{2-4}$-Alkylen-N($R^a$,$R^b$) oder gegebenenfalls durch bis zu 3-Substituenten aus der Gruppe von Hydroxy, Halogen, $C_{1-4}$-Alkyl, $C_{1-4}$-Alkoxy oder Nitro ringsubstituiertes Benzyl |
| Y | Methylen oder Aethylen |
| Z | Methylen , 0 oder S |
| $R^1$ | $C_{3-6}$-Cycloalkyl, N($R^c$,$R^d$), gegebenenfalls durch bis zu 3 Substituenten aus der Gruppe von Hydroxy, Halogen, $C_{1-4}$-Alkyl, $C_{1-4}$-Alkoxy oder Nitro substituiertes Phenyl oder gegebenenfalls fluoriertes $C_{1-4}$-Alkyl oder $C_{2-4}$-Alkenyl |
| $R^2$ | Wasserstoff, $C_{1-4}$-Alkyl oder, falls n = 0 ist, auch OH oder N($R^e$,$R^f$) |
| $R^a$ bis $R^g$ | Wasserstoff oder $C_{1-4}$-Alkyl sind, oder N($R^a$,$R^b$) ein 5- oder 6-gliedriger gesättigter Rest, gegebenenfalls mit einem zusätzlichen Heteroatom O oder N-$R^g$ ist, |

und von physiologisch verträglichen Salzen davon, dadurch gekennzeichnet, dass man

a) einen der Säure der Formel I entsprechenden $C_{1-4}$-Alkylester verseift oder

b) in einer Säure der Formel

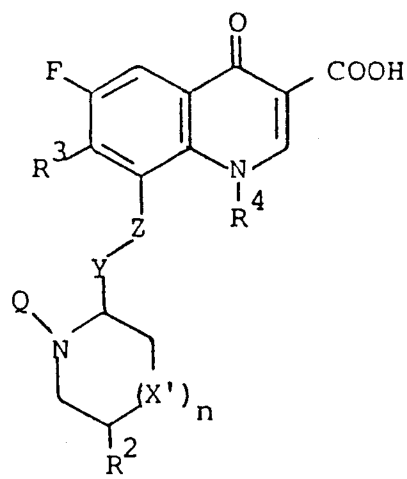

II

worin X' eine Gruppe N-R oder N-Q', Q und Q' Wasserstoff oder eine Stickstoffschutzgruppe. $R^3$ Halogen, und $R^4$ Wasserstoff sind oder $R^4$ die Bedeutung von $R^1$ hat, und $R^1$, $R^2$, R, Y, Z und n die obige Bedeutung haben,

allfällig vorhandene Stickstoffschutzgruppen abspaltet und das entstandene sekundäre Amin cyclisiert oder

c) eine Säure der Formel

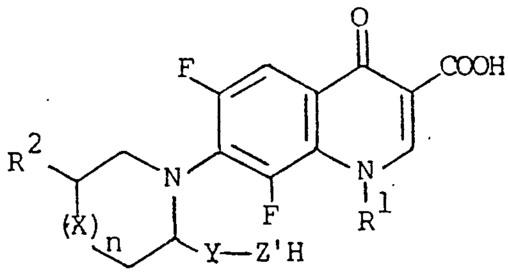

III

worin Z' Sauerstoff oder Schwefel ist und n, X, Y, $R^1$ und $R^2$ die obige Bedeutung haben, cyclisiert,

d) gewünschtenfalls eine erhaltene Verbindung der Formel I mit einem die Gruppe R bzw. die Gruppe $R^1$ einführenden Mittel umsetzt,

e) gewünschtenfalls die Hydroxygruppe $R^2$ in einer erhaltenen Verbindung der Formel I in eine Aminogruppe $N(R^e,R^f)$ umwandelt,

f) gewünschtenfalls den Vinylrest $R^1$ in einer erhaltenen Verbindung der Formel I in einen Cyclopropylrest umwandelt,

g) gewünschtenfalls eine erhaltene Verbindung der Formel I in Form eines Salzes isoliert.

**2.** Verfahren zur Herstellung von Chinolinderivaten der Formel

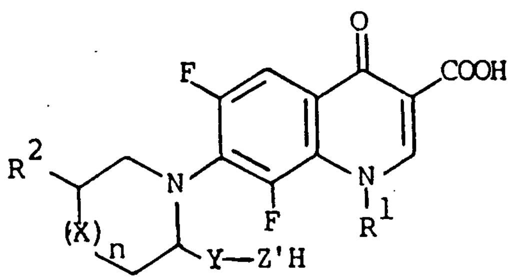

III

worin Z' Sauerstoff oder Schwefel ist und n, X, Y, $R^1$ und $R^2$ die gleiche Bedeutung wie in der Formel I haben,
dadurch gekennzeichnet, dass man einen der Säure der Formel III entsprechenden $C_{1-4}$-Alkylester verseift.

**3.** Verfahren nach Anspruch 1, worin n die Zahl 1, R Wasserstoff, $C_{1-4}$-Alkyl oder $C_{2-4}$-Alkylen-$N(R^a,R^b)$, und $R^2$ Wasserstoff ist.

**4.** Verfahren nach Anspruch 1 oder 2, worin R Wasserstoff, $C_{1-4}$-Alkyl, insbesondere Methyl oder Aethyl, oder ringsubstituiertes Benzyl, insbesondere p-Nitrobenzyl, ist.

**5.** Verfahren nach Anspruch 1 oder 2, worin Y Methylen und Z bzw. Z' Sauerstoff sind.

**6.** Verfahren nach Anspruch 1 oder 2, worin $R^1$ bzw. $R^4$ Wasserstoff, gegebenenfalls fluoriertes $C_{1-4}$-Alkyl, insbesondere Methyl, Aethyl oder 2-Fluoräthyl, $C_{3-6}$-Cycloalkyl, insbesondere Cyclopropyl; $N(R^c,R^d)$, insbesondere Methylamino, oder substituiertes Phenyl, insbesondere p-Fluorphenyl, ist.

**7.** Verfahren nach Anspruch 1 oder 2, worin $R^2$ Wasserstoff oder Hydroxy ist.

**8.** Verfahren nach Anspruch 4, worin R Wasserstoff oder Methyl ist.

**9.** Verfahren nach Anspruch 6, worin $R^1$ bzw. $R^4$ Aethyl, Cyclopropyl oder Methylamino ist.

**10.** Verfahren nach Anspruch 1, 8 oder 9 zur Herstellung einer Verbindung aus der Gruppe der folgenden
1-Cyclopropyl-6,8-difluor-1,4-dihydro-7-(2-hydroxymethyl-4-methyl-1-piperazinyl)-4-oxochinolin-3-carbonsäure

1-Aethyl-6-fluor-1,4,8,9,10,11,11a,12-octahydro-10-methyl-4-oxopyrazino[1',2':4,5)[1,4]oxazino[3,2-h]chinolin-3-carbonsäure

1-Aethyl-6-fluor-1,4,8,9,10,11,11a,12-octahydro-4-oxo-pyrazino[1',2':4,5][1,4]oxazino[3,2-h]chinolin-3-carbonsäure.

**11.** Verwendung einer Verbindung nach einem der Ansprüche 1 bis 10 zur Herstellung eines pharmazeutischen Präparats, insbesondere für die Therapie und Prophylaxe von bakteriellen Infektionen.

**Claims**

**Claims for the following Contracting States: BE, CH, DE, FR, GB, IT, LI, LU, NL, SE**

**1.** Quinoline derivatives of the formula

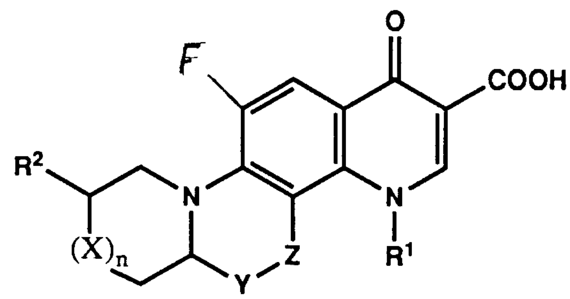

I

wherein

| | |
|---|---|
| n | is the number 1 or 0 |
| X | is a group N-R |
| R | is hydrogen, $C_{1-4}$-alkyl, $C_{2-4}$-alkylene-N($R^a$,$R^b$) or benzyl optionally ring-substituted by up to 3 substituents from the group of hydroxy, halogen, $C_{1-4}$-alkyl, $C_{1-4}$-alkoxy or nitro |
| Y | is methylene or ethylene |
| Z | is methylene, O or S |
| $R^1$ | is $C_{3-6}$-cycloalkyl, N($R^c$,$R^d$), phenyl optionally substituted by up to 3 substituents from the group of hydroxy, halogen, $C_{1-4}$-alkyl, $C_{1-4}$-alkoxy or nitro, or optionally fluorinated $C_{1-4}$-alkyl or $C_{2-4}$-alkenyl, |
| $R^2$ | is hydrogen, $C_{1-4}$-alkyl or, where n = 0, also OH or N($R^e$,$R^f$), |
| $R^a$ to $R^g$ | are hydrogen or $C_{1-4}$-alkyl or N($R^a$,$R^b$) is a 5- or 6-membered saturated residue optionally containing an additional hetero atom O or N-$R^g$, |

and physiologically compatible salts thereof.

**2.** Quinoline derivatives of the formula

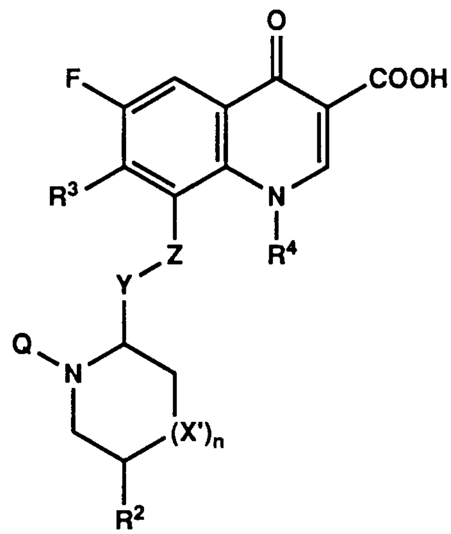

II

wherein X' is a group N-R or N-Q', Q and Q' are hydrogen or a nitrogen protecting group, $R^3$ is halogen and $R^4$ is hydrogen or $R^4$ has the same significance as $R^1$, and $R^1$, $R^2$, R, Y, Z and n have the same significance as in formula I.

**3.** Quinoline derivatives of the formula

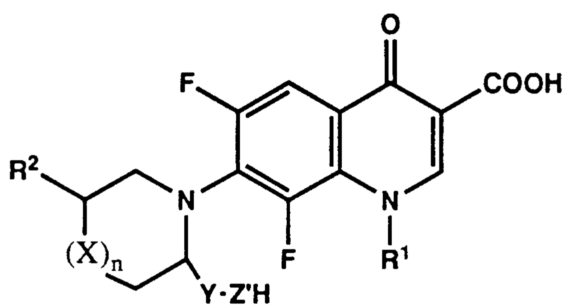

III

wherein Z' is oxygen or sulphur and n, X, Y, $R^1$ and $R^2$ have the same significance as in formula I.

**4.** Compounds according to claim 1 or 2, wherein n is the number 1, R is hydrogen, $C_{1-4}$-alkyl or $C_{2-4}$-alkylene-N($R^a$,$R^b$) and $R^2$ is hydrogen.

**5.** Compounds according to claim 1, 2 of 3, wherein R is hydrogen $C_{1-4}$-alkyl, especially methyl or ethyl, or ring-substituted benzyl, especially p-nitrobenzyl.

**6.** Compounds according to claim 1, 2 or 3, wherein Y is methylene and Z or Z' is oxygen.

**7.** Compounds according to claim 1, 2 or 3, wherein $R^1$ or $R^4$ is hydrogen, optionally fluorinated $C_{1-4}$-alkyl, especially methyl, ethyl or 2-fluoroethyl, $C_{3-6}$cycloalkyl, especially cyclopropyl; N($R^c$,$R^d$), especially methylamino, or substituted phenyl, especially p-fluorophenyl.

**8.** Compounds according to claim 1, 2 or 3, wherein $R^2$ is hydrogen or hydroxy.

**9.** Compounds according to claim 5, wherein R is hydrogen or methyl.

**10.** Compounds according to claim 7, wherein $R^1$ or $R^4$ is ethyl, cyclopropyl or methylamino.

**11.** A compound according to claim 1, 9 or 10 selected from the following group
1-cyclopropyl-6,8-difluoro-1,4-dihydro-7-(2-hydroxymethyl-4-methyl-1-piperazinyl)-4-oxoquinoline-3-carboxylic acid

1-ethyl-6-fluoro-1,4,8,9,10,11,11a,12-octahydro-10-methyl-4-oxopyrazino[1',2':4,5][1,4]oxazino[3,2-h]quinoline-3-carboxylic acid

1-ethyl-6-fluoro-1,4,8,9,10,11,11a,12-octahydro-4-oxopyrazino[1',2':4,5][1,4]oxazino[3,2-h]quinoline-3-carboxylic acid.

**12.** Compounds according to claims 1, 3 or 11 for use as therapeutically active substances, especially as antibacterially active substances.

**13.** A process for the manufacture of a compound according to claim 1, characterized by

a) saponifying a $C_{1-4}$-alkyl ester corresponding to the acid of formula I, or

b) cleaving off nitrogen protecting groups which may be present in an acid of the formula

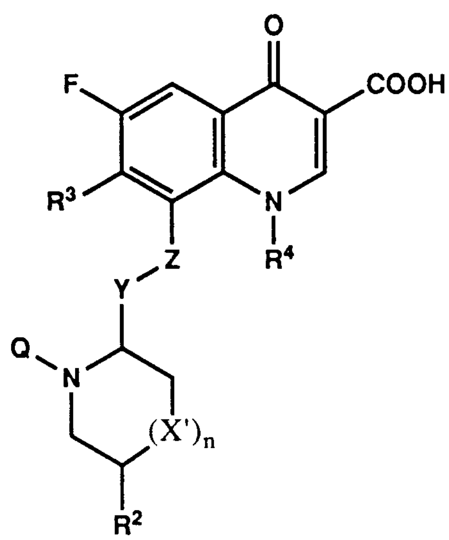

II

wherein X' is a group N-R or N-Q', Q and Q' are hydrogen or a nitrogen protecting group, $R^3$ is halogen and $R^4$ is hydrogen or $R^4$ has the significance of $R^1$, and $R^1$, $R^2$, R, Y, Z and n have the same significance as in formula I,

and cyclizing the resulting secondary amine, or

c) cyclizing an acid of the formula

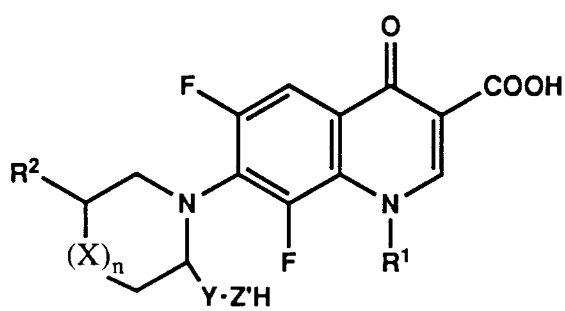

III

wherein Z' is oxygen or sulphur and n, X, Y, R' and $R^2$ have the significance as in formula I,

d) if desired, reacting a compound of formula I obtained with an agent which introduces the group R or the group $R^1$,

e) if desired, converting the hydroxy group $R^2$ in a compound of formula I obtained into an amino group $N(R^e,R^f)$,

f) if desired, converting the vinyl residue $R^1$ in a compound of formula I obtained into a cyclopropyl residue,

g) if desired, isolating a compound of formula I obtained in the form of a salt.

**14.** A process for the preparation of a compound according to claim 3, characterized by saponifying a $C_{1-4}$-alkyl ester corresponding to the acid of formula III.

**15.** A preparation based on a compound according to any one of claims 1, 3 or 11, especially for the therapy and prophylaxis of bacterial infections.

**16.** The use of a compound according to any one of claims 1, 3 or 11 for the manufacture of a preparation according to claim 15.

**Claims for the following Contracting State: AT**

**1.** A process for the manufacture of quinoline derivatives of the formula

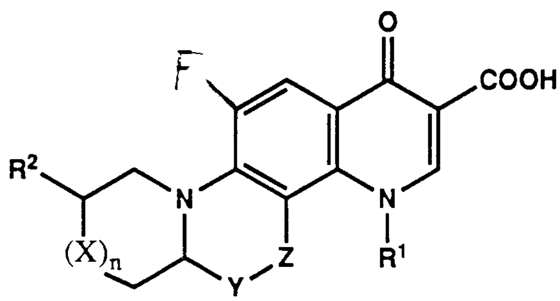

I

wherein

| | |
|---|---|
| n | is the number 1 or 0 |
| X | is a group N-R |
| R | is hydrogen, $C_{1-4}$-alkyl, $C_{2-4}$-alkylene-N($R^a$ ,$R^b$) or benzyl optionally ring-substituted by up to 3 substituents from the group of hydroxy, halogen, $C_{1-4}$-alkyl, $C_{1-4}$-alkoxy or nitro |
| Y | is methylene or ethylene |
| Z | is methylene, O or S |
| $R^1$ | is $C_{3-6}$-cycloalkyl, N($R^c$,$R^d$), phenyl optionally substituted by up to 3 substituents from the group of hydroxy, halogen, $C_{1-4}$-alkyl, $C_{1-4}$-alkoxy or nitro, or optionally fluorinated $C_{1-4}$-alkyl or $C_{2-4}$-alkenyl, |
| $R^2$ | is hydrogen, $C_{1-4}$-alkyl or, where n = 0, also OH or N($R^e$,$R^f$), |
| $R^a$ to $R^g$ | are hydrogen or $C_{1-4}$-alkyl or N($R^a$,$R^b$) is a 5- or 6-membered saturated residue optionally containing an additional hetero atom O or N-$R^g$, |

and physiologically compatible salts thereof, characterized by the following steps:

a) saponifying a $C_{1-4}$-alkyl ester corresponding to the acid of formula I, or

b) cleaving off nitrogen protecting groups which may be present in an acid of the formula

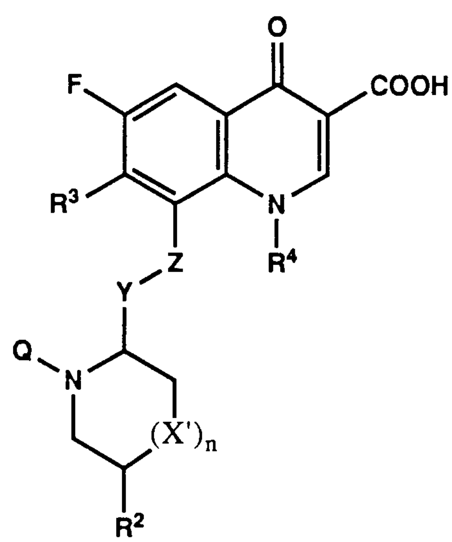

II

wherein X' is a group N-R or NQ', Q and Q' are hydrogen or a nitrogen protecting group, $R^3$ is

halogen and $R^4$ is hydrogen or $R^4$ has the significance of $R^1$, and $R^1$, $R^2$, R, Y, Z and n have the above significance,
and cyclizing the resulting secondary amine, or
c) cyclizing an acid of the formula

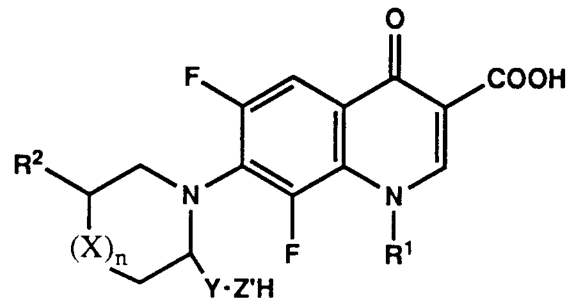

III

wherein Z' is oxygen or sulphur and n, X, Y, $R^1$ and $R^2$ have the above significance,
d) if desired, reacting a compound of formula I obtained with an agent which introduces the group R or the group $R^1$,
e) if desired, converting the hydroxy group $R^2$ in a compound of formula I obtained into an amino group $N(R^e,R^f)$,
f) if desired, converting the vinyl residue $R^1$ in a compound of formula I obtained into a cyclopropyl residue,
g) if desired, isolating a compound of formula I obtained in the form of a salt.

**2.** A process for the preparation of quinoline derivatives of the formula

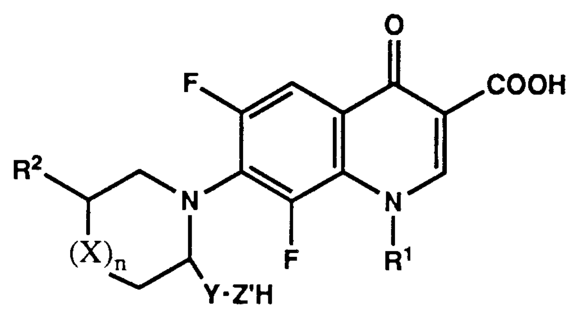

III

wherein Z' is oxygen or sulphur and n, X, Y, $R^1$ and $R^2$ have the same significance as in formula I, characterized by saponifying a $C_{1-4}$-alkyl ester corresponding to the acid of formula III.

**3.** A process according to claim 1, wherein n is the number 1, R is hydrogen, $C_{1-4}$-alkyl or $C_{2-4}$-alkylene-$N(R^a,R^b)$ and $R^2$ is hydrogen.

**4.** A process according to claim 1 or 2, wherein R is hydrogen, $C_{1-4}$-alkyl, especially methyl or ethyl, or ring-substituted benzyl, especially p-nitrobenzyl.

**5.** A process according to claim 1 or 2, wherein Y is methylene and Z or Z' is oxygen.

**6.** A process according to claim 1 or 2, wherein $R^1$ or $R^4$ is hydrogen, optionally fluorinated $C_{1-4}$-alkyl, especially methyl, ethyl or 2-fluoroethyl, $C_{3-6}$-cycloalkyl, especially cyclopropyl, $N(R^c,R^d)$, especially methylamino, or substituted phenyl, especially p-fluorophenyl.

**7.** A process according to claim 1 or 2, wherein $R^2$ is hydrogen or hydroxy.

**8.** A process according to claim 4, wherein R is hydrogen or methyl.

**9.** A process according to claim 6, wherein $R^1$ or $R^4$ is ethyl, cyclopropyl or methylamino.

**10.** A process according to claim 1, 8 or 9 for the manufacture of a compound from the following group
1-cyclopropyl-6,8-difluoro-1,4-dihydro-7-(2-hydroxymethyl-4-methyl-1-piperazinyl)-4-oxoquinoline-3-carboxylic acid
1-ethyl-6-fluoro-1,4,8,9,10,11,11a,12-octahydro-10-methyl-4-oxopyrazino[1',2':4,5](1,4]oxazino[3,2-h]quinoline-3-carboxylic acid
1-ethyl-6-fluoro-1,4,8,9,10,11,11a,12-octahydro-4-oxopyrazino[1',2':4,5][1,4]oxazino[3,2-h]quinoline-3-carboxylic acid.

**11.** The use of a compound according to any one of claims 1 to 10 for the manufacture of a pharmaceutical preparation, especially for the therapy and prophylaxis of bacterial infections.

**Revendications**
**Revendications pour les Etats contractants suivants: BE, CH, DE, FR, GB, IT, LI, LU, NL, SE**

**1.** Dérivés de quinoléine de formule

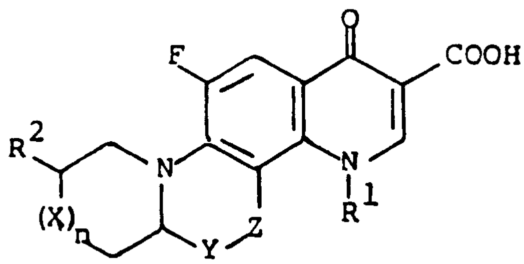

I

où

| | |
|---|---|
| n | est le nombre 1 ou 0 |
| X | est un groupe N-R |
| R | représente l'hydrogène, un groupe alkyle en $C_{1-4}$, un groupe alkylène($C_{2-4}$)-N($R^a$,$R^b$) ou un groupe benzyle éventuellement substitué sur le cycle par jusqu'à 3 substituants choisis parmi les radicaux hydroxy, halogène, alkyle en $C_{1-4}$, alcoxy en $C_{1-4}$ ou nitro |
| Y | est un radical méthylène ou éthylène |
| Z | est un radical méthylène, O ou S |
| $R^1$ | est un groupe cycloalkyle en $C_{3-6}$, N($R^c$,$R^d$), un groupe phényle éventuellement substitué par jusqu'à 3 substituants choisis parmi les radicaux hydroxy, halogène, alkyle en $C_{1-4}$, alcoxy en $C_{1-4}$ ou nitro ou un groupe alkyle en $C_{1-4}$ ou alcényle en $C_{2-4}$ éventuellement fluorés |
| $R^2$ | est l'hydrogène, un groupe alkyle en $C_{1-4}$ ou, dans le cas où n = 0, également OH ou N($R^e$,$R^f$) |
| $R^a$ à $R^g$ | sont l'hydrogène ou des groupes alkyle en $C_{1-4}$, ou N($R^a$,$R^b$) est un reste saturé à 5 ou 6 chaînons, éventuellement avec un hétéroatome supplémentaire 0 ou N-$R^g$, |

et leurs sels physiologiquement acceptables.

**2.** Dérivés de quinoléine de formule

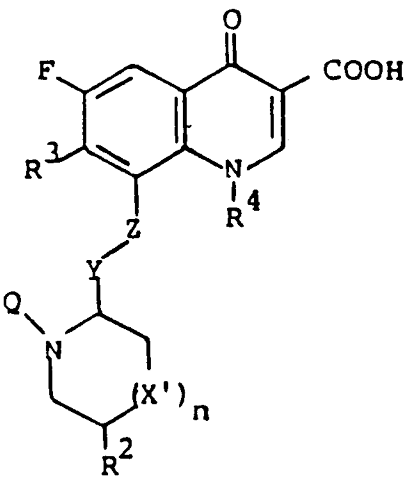

II

où X' est un groupe N-R ou N-Q', Q et Q' sont de l'hydrogène ou un groupe protecteur de l'azote, $R^3$ est un halogène, et $R^4$ est de l'hydrogène ou $R^4$ a la même signification que $R^1$ et $R^1$, $R^2$, R, Y, Z et n ont la même signification que dans la formule I.

**3.** Dérivés de quinoléine de formule

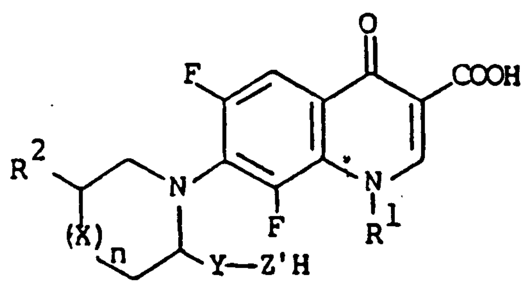

III

où Z' est de l'oxygène ou du soufre et n, X, Y, $R^1$ et $R^2$ ont la même signification que dans la formule I.

**4.** Composés selon l'une des revendications 1 ou 2, dans lesquels n est le nombre 1, R est de l'hydrogène, un groupe alkyle en $C_{1-4}$ ou un groupe alkylène($C_{2-4}$)-N($R^a$,$R^b$) et $R^2$ est l'hydrogène.

**5.** Composés selon l'une quelconque des revendications 1, 2 ou 3, dans lesquels R est l'hydrogène, un groupe alkyle en $C_{1-4}$, en particulier méthyle ou éthyle, ou un groupe benzyle substitué sur le cycle, en particulier p-nitrobenzyle.

**6.** Composés selon l'une quelconque des revendications 1, 2 ou 3, dans lesquels Y est un groupe méthylène et Z et Z' sont de l'oxygène.

**7.** Composés selon l'une quelconque des revendications 1, 2 ou 3, dans lesquels $R^1$ et $R^4$ respectivement sont de l'hydrogène, un groupe alkyle en $C_{1-4}$ éventuellement fluoré, en particulier méthyle, éthyle ou 2-fluoroéthyle, un groupe cycloalkyle en $C_{3-6}$, en particulier cyclopropyle, N($R^c$,$R^d$), en particulier méthylamino, ou un groupe phényle substitué, en particulier p-fluorophényle.

**8.** Composés selon l'une quelconque des revendications 1, 2 ou 3, dans lesquels $R^2$ est l'hydrogène ou un radical hydroxy.

**9.** Composés selon la revendication 5, dans lesquels R est l'hydrogène ou un groupe méthyle.

**10.** Composés selon la revendication 7, dans lesquels $R^1$ et $R^4$ respectivement sont un groupe éthyle, cyclopropyle ou méthylamino.

**11.** Composés selon l'une quelconque des revendications 1, 9 ou 10, choisis parmi les suivants:
acide 1-cyclopropyl-6,8-difluoro-1,4-dihydro-7-(2-hydroxyméthyl-4-méthyl-1-pipérazinyl)-4-oxoquinolyl-3-carboxylique
acide 1-éthyl-6-fluoro-1,4,8,9,10,11,11a,12-octahydro-10-méthyl-4-oxopyrazino[1',2':4,5][1,4]oxazino-[3,2-h]-quinolyl-3-carboxylique
acide 1-éthyl-6-fluoro-1,4,8,9,10,11,11a,12-octahydro-4-oxopyrazino[1',2':4,5][1,4]oxazino[3,2-h]-quinolyl-3-carboxylique.

**12.** Composés selon l'une quelconque des revendications 1, 3 ou 11, pour une utilisation comme constituant à activité thérapeutique, en particulier antibactérienne.

**13.** Procédé pour la préparation d'un composé selon la revendication 1, caractérisé en ce qu'on
a) saponifie un ester d'alkyle en $C_{1-4}$ correspondant à l'acide de formule I, ou
b) élimine les groupes protecteurs de l'azote éventuellement présents dans un acide de formule

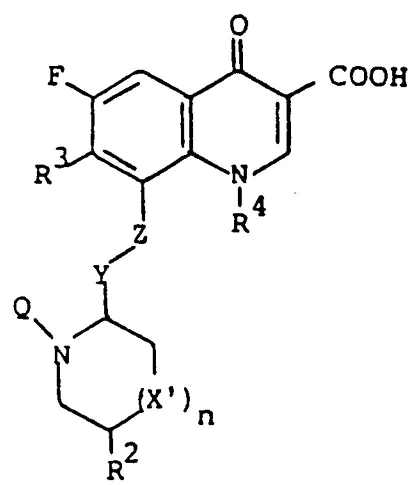

II

où X' est un groupe N-R ou N-Q', Q et Q' sont de l'hydrogène ou un groupe protecteur de l'azote, $R^3$ est un halogène, et $R^4$ est de l'hydrogène ou $R^4$ a la signification de $R^1$, et $R^1$, $R^2$, R, Y, Z et n ont la même signification que dans la formule I,
et cyclise l'amine secondaire résultante, ou
c) cyclise un acide de formule

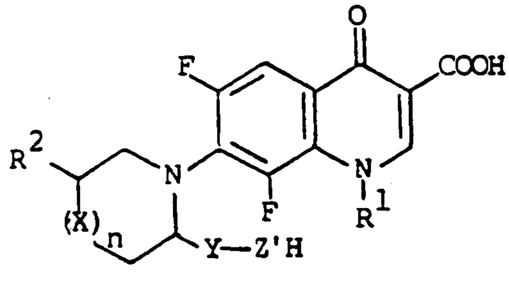

III

où Z' est l'oxygène ou le soufre et n, X, Y, $R^1$ et $R^2$ ont la même signification que dans la formule I,
d) éventuellement fait réagir un composé obtenu de formule I avec un agent introduisant le groupe R ou le groupe $R^1$,
e) éventuellement convertit le groupe hydroxy $R^2$ dans un composé obtenu de formule I en un groupe amino $N(R^e,R^f)$,
f) éventuellement convertit le reste vinyle $R^1$ dans un composé obtenu de formule I en un reste cyclopropyle,
g) éventuellement isole un composé obtenu de formule I sous forme d'un sel.

**14.** Procédé pour la préparation d'un composé selon la revendication 3, caractérisé en ce qu'on saponifie un ester d'alkyle en $C_{1-4}$ correspondant à l'acide de formule III.

**15.** Préparation à base d'un composé selon l'une quelconque des revendications 1, 3 ou 11, en particulier pour la thérapie ou la prophylaxie des infections bactériennes.

**16.** Utilisation d'un composé selon l'une quelconque des revendications 1, 3 ou 11 pour la production d'une préparation selon la revendication 15.

**Revendications pour l'Etat contractant suivant: AT**

**1.** Procédé pour la préparation de dérivés de quinoléine de formule

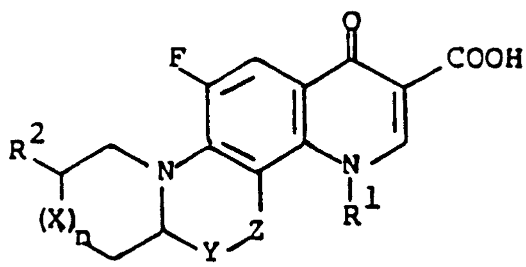

I

où

| | |
|---|---|
| n | est le nombre 1 ou 0 |
| X | est un groupe N-R |
| R | représente l'hydrogène, un groupe alkyle en $C_{1-4}$, un groupe alkylène($C_{2-4}$)-N($R^a$,$R^b$) ou un groupe benzyle éventuellement substitué sur le cycle par jusqu'à 3 substituants choisis parmi les radicaux hydroxy, halogène, alkyle en $C_{1-4}$, alcoxy en $C_{1-4}$ ou nitro |
| Y | est un radical méthylène ou éthylène |
| Z | est un radical méthylène, O ou S |
| $R^1$ | est un groupe cycloalkyle en $C_{3-6}$, N($R^c$,$R^d$), un groupe phényle éventuellement substitué par jusqu'à 3 substituants choisis parmi les radicaux hydroxy, halogénè, alkyle en $C_{1-4}$, alcoxy en $C_{1-4}$ ou nitro ou un groupe alkyle en $C_{1-4}$ ou alcényle en $C_{2-4}$ éventuellement fluorés |
| $R^2$ | est l'hydrogène, un groupe alkyle en $C_{1-4}$ ou, dans le cas où n = 0, également OH ou N($R^e$,$R^f$) |
| $R^a$ à $R^g$ | sont l'hydrogène ou des groupes alkyle en $C_{1-4}$, ou N($R^a$,$R^b$) est un reste saturé à 5 ou 6 chaînons, éventuellement avec un hétéroatome supplémentaire O ou N-$R^g$, |

et de leurs sels physiologiquement acceptables, caractérisé en ce qu'on

a) saponifie un ester d'alkyle en $C_{1-4}$ correspondant à l'acide de formule I, ou

b) élimine les groupes protecteurs de l'azote éventuellement présents dans un acide de formule

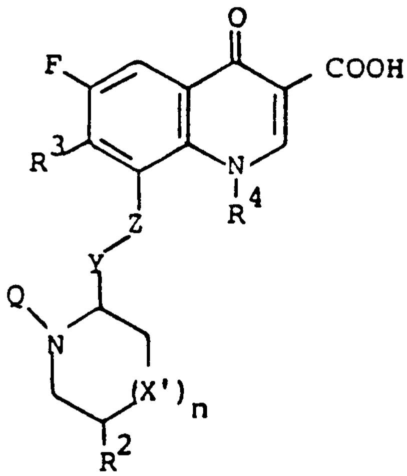

II

où X' est un groupe N-R ou N-Q', Q et Q' sont de l'hydrogène ou un groupe protecteur de l'azote, $R^3$ est un halogène, et $R^4$ est de l'hydrogène ou $R^4$ a la signification de $R^1$ et $R^1$, $R^2$, R, Y, Z et n ont la même signification que dans la formule I,
et cyclise l'amine secondaire résultante, ou
c) cyclise un acide de formule

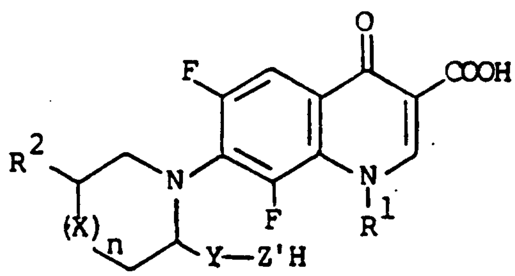

III

où Z' est l'oxygène ou le soufre et n, X, Y, $R^1$ et $R^2$ ont la même signification que dans la formule I,
d) éventuellement fait réagir un composé obtenu de formule I avec un agent introduisant le groupe R ou le groupe $R^1$,
e) éventuellement convertit le groupe hydroxy $R^2$ dans un composé obtenu de formule I en un groupe amino $N(R^e,R^f)$,
f) éventuellement convertit le reste vinyle $R^1$ dans un composé obtenu de formule I en un reste cyclopropyle,
g) éventuellement isole un composé obtenu de formule I sous forme d'un sel.

**2.** Procédé pour la préparation de dérivés de quinoléine de formule

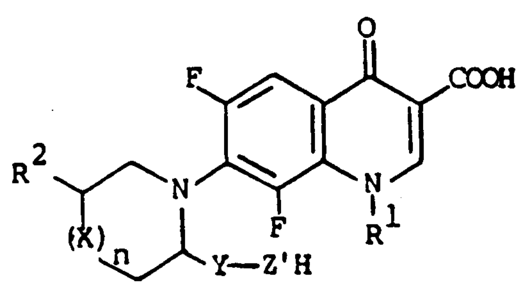

III

où Z' est de l'oxygène ou du soufre et n, X, Y, $R^1$ et $R^2$ ont la même signification que dans la formule I,
caractérisé en ce qu'on saponifie un ester d'alkyle en $C_{1-4}$ correspondant à l'acide de formule III.

**3.** Procédé selon la revendication 1, dans lequel n est le nombre 1, R est l'hydrogène, un groupe alkyle en $C_{1-4}$ ou un groupe alkylène($C_{2-4}$)-N($R^a$,$R^b$), et $R^2$ est l'hydrogène.

**4.** Procédé selon l'une des revendications 1 ou 2, dans lequel R est l'hydrogène, un groupe alkyle en $C_{1-4}$, en particulier méthyle ou éthyle, ou un groupe benzyle substitué sur le cycle, en particulier p-nitrobenzyle.

**5.** Procédé selon l'une des revendications 1 ou 2, dans lequel Y est le groupe méthylène et Z ou Z' respectivement est de l'oxygène.

**6.** Procédé selon l'une des revendications 1 ou 2, dans lequel $R^1$ ou $R^4$ est de l'hydrogène, un groupe alkyle en $C_{1-4}$ éventuellement fluoré, en particulier méthyle, éthyle ou 2-fluoroéthyle, un groupe cycloalkyle en $C_{3-6}$, en particulier cyclopropyle, N($R^c$,$R^d$), en particulier méthylamino, ou un groupe phényle substitué, en particulier p-fluorophényle.

**7.** Procédé selon l'une des revendications 1 ou 2, dans lequel $R^2$ est de l'hydrogène ou un radical hydroxy.

**8.** Procédé selon la revendication 4, dans lequel R est de l'hydrogène ou un groupe méthyle.

**9.** Procédé selon la revendication 6, dans lequel $R^1$ ou $R^4$ respectivement est un groupe éthyle, cyclopropyle ou méthylamino.

**10.** Procédé selon l'une quelconque des revendications 1, 8 ou 9 pour la préparation d'un composé choisi parmi les suivants:
acide 1-cyclopropyl-6,8-difluoro-1,4-dihydro-7-(2-hydroxyméthyl-4-méthyl-1-pipérazinyl)-4-oxoquinolyl-3-carboxylique
acide 1-éthyl-6-fluoro-1,4,8,9,10,11,11a,12-octahydro-10-méthyl-4-oxopyrazino[1',2':4,5][1,4]oxazino-[3,2-h]quinolyl-3-carboxylique
acide 1-éthyl-6-fluoro-1,4,8,9,10,11,11a,12-octahydro-4-oxopyrazino[1',2':4,5][1,4]oxazino[3,2-h]-quinolyl-3-carboxylique.

**11.** Utilisation d'un composé selon l'une quelconque des revendications 1 à 10 pour la production d'une préparation pharmaceutique, en particulier pour la thérapie et la prophylaxie des infections bactériennes.